# EUROPEAN PATENT APPLICATION

(11) **EP 1 541 138 A1**
(43) Date of publication of application: **15.06.2005**
(21) Application number: 03028139.8
(22) Date of filing: 05.12.2003
(51) Int. Cl.: A61K 31/165, A61P 25/08

(54) **Novel use of peptide compounds for treating status epilepticus or related conditions**

(71) Applicant: SCHWARZ PHARMA AG, 40789 Monheim (DE)
(72) Inventor: Rudd, David Ph.D., 27705 North Carolina (US); Stöhr, Thomas Dr.rer.nat., 40789 Monheim (DE)
(74) Representative: Weiss, Wolfgang, Dipl.-Chem. Dr.

(57) **Abstract**

The present invention is directed to the novel use of a class of peptide compounds for treating *status epilepticus* or related conditions, e.g. acute repetitive seizures, seizure clusters, etc.

## Description

The present invention is directed to the novel use of a class of peptide compounds for treating *status epilepticus* or related conditions, e.g. acute repetitive seizures, seizure clusters, etc.

Certain peptides are known to exhibit central nervous system (CNS) activity and are useful in the treatment of epilepsy, nervous anxiety, psychosis and insomnia. These peptides which are described in the U.S. Patent No. 5,378,729 have the Formula (Ia): 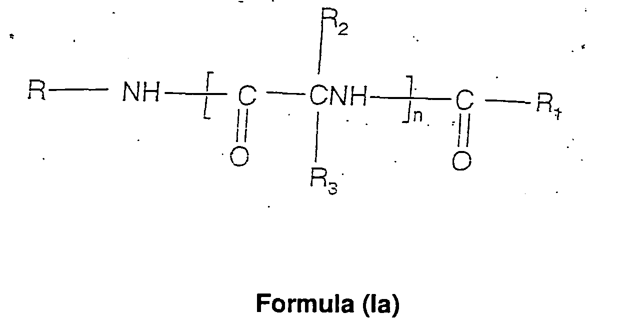 wherein
R is hydrogen, lower alkyl, lower alkenyl, lower alkynyl, aryl, aryl lower alkyl, heterocyclic, heterocyclic lower alkyl, lower alkyl heterocyclic, lower cycloalkyl, lower cycloalkyl lower alkyl, and R is unsubstituted or is substituted with at least one electron withdrawing group or electron donating group;
R₁ is hydrogen or lower alkyl, lower alkenyl, lower alkynyl, aryl lower alkyl, aryl, heterocyclic lower alkyl, heterocyclic, lower cycloalkyl, lower cycloalkyl lower alkyl, each unsubstituted or substituted with an electron donating group or an electron withdrawing group; and
R₂ and R₃ are independently hydrogen, lower alkyl, lower alkenyl, lower alkynyl, aryl lower alkyl, aryl, heterocyclic, heterocyclic lower alkyl, lower alkyl heterocyclic, lower cycloalkyl, lower cycloalkyl lower alkyl, or Z-Y wherein R₂ and R₃ may be unsubstituted or substituted with at least one electron withdrawing group or electron donating group;
Z is O, S, S(O)ₐ, NR₄, PR₄ or a chemical bond;
Y is hydrogen, lower alkyl, aryl, aryl lower alkyl, lower alkenyl, lower alkynyl, halo, heterocyclic, heterocyclic lower alkyl, and Y may be unsubstituted or substituted with an electron donating group or an electron withdrawing group, provided that when Y is halo, Z is a chemical bond, or
ZY taken together is NR₄NR₅R₇, NR₄OR₅, ONR₄R₇, OPR₄R₅, PR₄OR₅, SNR₄R₇, NR₄SR₇, SPR₄R₅ or PR₄SR₇, NR₄PR₅R₆ or PR₄NR₅R₇, 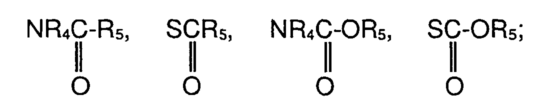
R₄, R₅ and R₆ are independently hydrogen, lower alkyl, aryl, aryl lower alkyl, lower alkenyl, or lower alkynyl, wherein R₄, R₅ and R₆ may be unsubstituted or substituted with an electron withdrawing group or an electron donating group; and
R₇ is R₆ or COOR₈ or COR₈;
R₈ is hydrogen or lower alkyl, or aryl lower alkyl, and the aryl or alkyl group may be unsubstituted or substituted with an electron withdrawing group or an electron donating group; and
n is 1-4; and
a is 1-3.

U.S. Patent No. 5,773,475 also discloses additional compounds useful for treating epilepsy, nervous anxiety, psychosis and insomnia. These compounds are N-benzyl-2-amino-3-methoxy-proprionamides having the Formula (IIa): 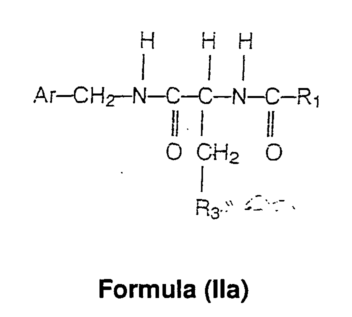 wherein
Ar is aryl which is unsubstituted or substituted with halo; R₃ is lower alkoxy; and R₁ is methyl.

WO 02/074297 relates to the use of a compound according to Formula (IIa) wherein Ar is phenyl which may be substituted by at least one halo, R₃ is lower alkoxy containing 1-3 carbon atoms and R₁ is methyl for the preparation of pharmaceutical compositions useful for the treatment of allodynia related to peripheral neuropathic pain.

The patents US 5.378.729 and US 5.773.475 are hereby incorporated by reference. However, neither of these patents describes the use of these compounds for the treatment of *status epilepticus.*

Seizures are the consequence of a paroxysmal brain dysfunction related to excessive neuronal activity that leads to an alteration of behaviour or consciousness. Epilepsy represents the recurrence of two or more unprovoked seizures and represents a chronic brain disease. About 0.5% of the population suffers epilepsy, and up to 10% of the population will suffer at least one seizure during their life-time.

There are two major types of seizures: partial or focal seizures, which originate in a location in the brain, but can spread in the course of the event; and generalized seizures, which can affect both hemispheres simultaneously. Partial seizures are manifested in multiple ways depending on the area that is affected (confusion, automatic body movements, hallucinations, etc), and if they spread in the brain can end up in a generalized tonic-clonic event (a convulsion). There are several types of generalized seizures: convulsive (tonic-clonic, tonic, clonic, myoclonic) and non-convulsive (absences, atonic). Typically all kinds of seizures last a few minutes, usually less than five minutes.

*Status epilepticus* is currently defined as a seizure that lasts for 30 or more minutes, or a series of consecutive seizures that occur for 30 or more minutes during which the subject does not completely recover consciousness. Many clinicians and many recent major research articles, however, consider a patient to be in status if seizures last more than 10 minutes. There are two main types of status: generalized (convulsive and non-convulsive) and focal. The generalized convulsive status is the most severe type and is associated with high morbidity and mortality. *Status epilepticus* can occur in patients with prior epilepsy diagnosis. However, the onset of status is more frequent in subjects without previous epilepsy and is often related to a severe and acute brain disease (for example, an encephalitis or a stroke).

*Status epilepticus* or related conditions represent an emergency and pharmalogical treatment should preferably be carried out using intravenous medication. Drugs used for initial treatment are intravenous benzodiazepines (for example diazepam, lorazepam), phenytoin, fosphenytoin and phenobarbital. Intravenous valproic acid has also been used. Rectal or intramuscular administration routes may also be used. Despite these first line treatments, over 40% of the subjects will not respond. Under these circumstances, pharmacological coma is needed to treat status (pentobarbital, propofol, high dose of midazolam or other benzodiazepines).

Current antiepileptic drugs are believed to work through diverse mechanisms of action: altering neuronal impulse propagation via interaction with voltage gated sodium-, calcium- or potassium channels or affecting neural transmission by either potentiating inhibitory GABA systems or by inhibition of excitatory glutamate systems.

(R)-2-acetamide-N-benzyl-3-methoxypropionamide (SPM 927, also called Harkoseride) is a functionalized amino acid initially synthesized as an anticonvulsant. SPM 927 appears to be more potent and effective as compared to other clinically effective anticonvulsant drugs (phenytoin, carbamazepine) when it was evaluated in several anticonvulsant animal models.

The use of compounds of Formula (Ib) or/and Formula (IIb) for treatment of *status epilepticus* has not been reported. Thus, the present invention concerns the use of said compounds of Formulae (Ib) or/and (IIb) for the preparation of a pharmaceutical composition for the treatment of *status epilepticus* or related conditions, e.g. acute repetitive seizures, seizure clusters, etc.

Surprisingly, in animal models of self-sustaining *status epilepticus* (SSSE), it was found that compounds of Formula (Ib) or/and (IIb), particularly SPM927, potently reduced cumulative SSSE duration after brief electrical stimulation of the perforant path in two independent experiments. SPM 927 effect in reducing the number of spikes was significantly more potent than that of fosphenytoin. Moreover, SPM 927 was somewhat more efficacious when compared to diazepam and phenytoin. SPM 927 prevented spontaneous generalized tonic clonic seizures (GTCS) in the cobalt/homocysteine model of SSSE. These results suggest that i.v. SPM 927 is useful for the treatment of *status epilepticus.*

Another surprising property of the components of Formula (Ib) or/and (lib), especially SPM 927, is the ability of these compounds to protect neuronal tissue, in particular hippocampal tissue, against damage caused by GTCS. The hippocampus is exceptionally vulnerable to damage caused by the pathological neuronal discharges during GTCS. Thus, the compounds of Formula (Ib) or/and (IIb), particularly SPM 927, are useful for the neuroprotective treatment of effects before/during acute seizures occurring in particular during *status epilepticus* or related conditions, or during chronic seizure disorders (e.g. epilepsy) to reduce brain damage, short term memory loss, cognitive decline, additional seizures (anti-epileptogenesis), etc.

The compounds of the present invention show a unique profile in animal models for epilepsy in comparison with widely used antiepileptic drugs. The compounds of the present invention are active in an animal model which is used for the identification of compounds limiting seizure spread. Furthermore, the compounds were found to be active in an animal model which allows for detection of compounds acting against treatment resistant partial seizures. Thus, the compounds of Formula (Ib) or/and (IIb), particularly SPM927, are useful for treatment of complex partial seizures, treatment of partial seizures resistant to other treatments, seizure spread or generalized tonic-clonic seizures.

The compounds of Formula (Ib) or/and (IIb), in particular SPM 927, are well tolerated, which is an advantage over other commonly used therapeutics for treatment of *status epilepticus* or related conditions.

The mode of action of the compounds (Ib) or/and (IIb) differs from that of common antiepileptic drugs. Ion channels are not affected by the compounds of the present invention, whereas GABA-induced currents are potentiated, but no direct interaction with any known GABA receptor subtype is observed. Glutamate induced currents are attenuated but the compounds do not directly interact with any known glutamate receptor subtype.

Thus, a compound according to the invention useful for the treatment of *status epilepticus* or related conditions has the general Formula (Ib) 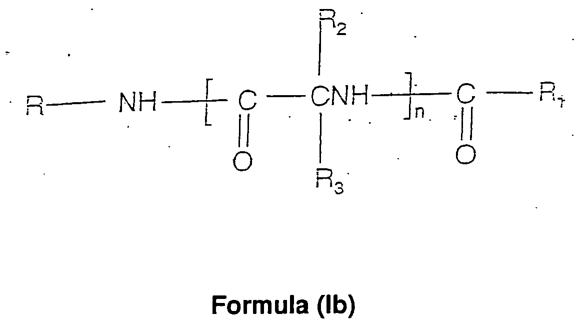 wherein
R is hydrogen, lower alkyl, lower alkenyl, lower alkynyl, aryl, aryl lower alkyl, heterocyclic, heterocyclic lower alkyl, lower alkyl heterocyclic, lower cycloalkyl or lower cycloalkyl lower alkyl, and R is unsubstituted or is substituted with at least one electron withdrawing group, or electron donating group;
R₁ is hydrogen or lower alkyl, lower alkenyl, lower alkynyl, aryl lower alkyl, aryl, heterocyclic lower alkyl, lower alkyl heterocyclic, heterocyclic, lower cycloalkyl, lower cycloalkyl lower alkyl, each unsubstituted or substituted with an electron donating group or an electron withdrawing group;
and
R₂ and R₃ are independently hydrogen, lower alkyl, lower alkenyl, lower alkynyl, aryl lower alkyl, aryl, halo, heterocyclic, heterocyclic lower alkyl, lower alkyl heterocyclic, lower cycloalkyl, lower cycloalkyl lower alkyl, or Z-Y wherein R₂ and R₃ may be unsubstituted or substituted with at least one electron withdrawing group or electron donating group;
Z is 0, S, S(O)ₐ, NR₄, NR'₆, PR₄ or a chemical bond;
Y is hydrogen, lower alkyl, aryl, aryl lower alkyl, lower alkenyl, lower alkynyl, halo, heterocyclic, heterocyclic lower alkyl, lower alkyl heterocyclic and Y may be unsubstituted or substituted with an electron donating group or an electron withdrawing group, provided that when Y is halo, Z is a chemical bond, or
ZY taken together is NR₄NR₅R₇, NR₄OR₅, ONR₄R₇, OPR₄R₅, PR₄OR₅, SNR₄R₇, NR₄SR₇, SPR₄R₅, PR₄SR₇, NR₄PR₅R₆, PR₄NR₅R₇ or N⁺R₅R₆R₇, 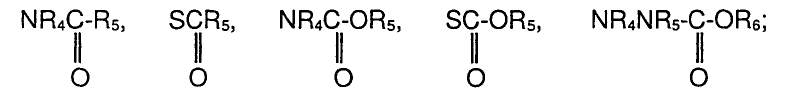
R'₆ is hydrogen, lower alkyl, lower alkenyl, or lower alkenyl which may be unsubstituted or substituted with an electron withdrawing group or electron donating group;
R₄, R₅ and R₆ are independently hydrogen, lower alkyl, aryl, aryl lower alkyl, lower alkenyl, or lower alkynyl, wherein R₄, R₅ and R₆ may independently be unsubstituted or substituted with an electron withdrawing group or an electron donating group;
R₇ is R₆ or COOR₈ or COR₈, which R₇ may be unsubstituted or substituted with an electron withdrawing group or an electron donating group;
R₈ is hydrogen or lower alkyl, or aryl lower alkyl, and the aryl or alkyl group may be unsubstituted or substituted with an electron withdrawing group or an electron donating group; and
n is 1-4; and
a is 1-3.

Furthermore a compound according to the invention has the general Formula (IIb) 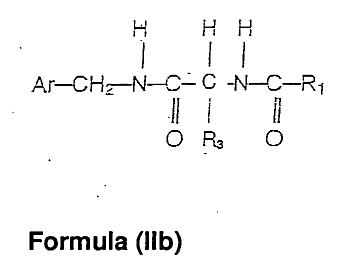 wherein
Ar is aryl, especially phenyl, which is unsubstituted or substituted with at least one halo; R₃ is -CH₂-Q, wherein Q is lower alkoxy; and R₁ is lower alkyl, especially methyl.

The present invention is also directed to the preparation of pharmaceutical compositions comprising a compound according to Formula (Ib) and/or Formula (IIb) useful for the treatment of *status epilepticus.*

The compounds of Formula (Ia) are described in U.S. Patent No. 5,378,729, the contents of which are incorporated by reference.

The "lower alkyl" groups when used alone or in combination with other groups, are lower alkyl containing from 1 to 6 carbon atoms, especially 1 to 3 carbon atoms, and may be straight chain or branched. These groups include methyl, ethyl, propyl, isopropyl, butyl, isobutyl, tertiary butyl, amyl, hexyl, and the like.

The "lower alkoxy" groups are lower alkoxy containing from 1 to 6 carbon atoms, especially 1 to 3 carbon atoms, and may be straight chain or branched. These groups include methoxy, ethoxy, propoxy, butoxy, isobutoxy, tert-butoxy, pentoxy, hexoxy and the like.

The "aryl lower alkyl" groups include, for example, benzyl, phenethyl, phenpropyl, phenisopropyl, phenbutyl, diphenylmethyl, 1,1-diphenylethyl, 1,2-diphenylethyl, and the like.

The term "aryl", when used alone or in combination, refers to an aromatic group which contains from 6 up to 18 ring carbon atoms and up to a total of 25 carbon atoms and includes the polynuclear aromatics. These aryl groups may be monocyclic, bicyclic, tricyclic or polycyclic and are fused rings. A polynuclear aromatic compound as used herein, is meant to encompass bicyclic and tricyclic fused aromatic ring systems containing from 10-18 ring carbon atoms and up to a total of 25 carbon atoms. The aryl group includes phenyl, and the polynuclear aromatics e.g., naphthyl, anthracenyl, phenanthrenyl, azulenyl and the like. The aryl group also includes groups like ferrocyenyl.

"Lower alkenyl" is an alkenyl group containing from 2 to 6 carbon atoms and at least one double bond. These groups may be straight chained or branched and may be in the Z or E form. Such groups include vinyl, propenyl, 1-butenyl, isobutenyl, 2-butenyl, 1-pentenyl, (Z)-2-pentenyl, (E)-2-pentenyl, (Z)-4-methyl-2-pentenyl, (E)-4-methyl-2-pentenyl, pentadienyl, e.g., 1, 3 or 2,4-pentadienyl, and the like.

The term "lower alkynyl" is an alkynyl group containing 2 to 6 carbon atoms and may be straight chained as well as branched. It includes such groups as ethynyl, propynyl, 1-butynyl, 2-butynyl, 1-pentynyl, 2-pentynyl, 3-methyl-1-pentynyl, 3-pentynyl, 1-hexynyl, 2-hexynyl, 3-hexynyl and the like.

The term "lower cycloalkyl" when used alone or in combination is a cycloalkyl group containing from 3 to 18 ring carbon atoms and up to a total of 25 carbon atoms. The cycloalkyl groups may be monocyclic, bicyclic, tricyclic, or polycyclic and the rings are fused. The cycloalkyl may be completely saturated or partially saturated. Examples include cyclopropyl, cyclobutyl, cyclopentyl, cyclohexyl, cycloheptyl, cyclooctyl, cyclodecyl, cyclohexenyl, cyclopentenyl, cyclooctenyl, cycloheptenyl, decalinyl, hydroindanyl, indanyl, fenchyl, pinenyl, adamantyl, and the like. Cycloalkyl includes the cis or trans forms. Furthermore, the substituents may either be in endo or exo positions in the bridged bicyclic systems.

The term "electron-withdrawing and electron donating" refer to the ability of a substituent to withdraw or donate electrons, respectively, relative to that of hydrogen if the hydrogen atom occupied the same position in the molecule. These terms are well understood by one skilled in the art and are discussed in Advanced Organic Chemistry, by J. March, John Wiley and Sons, New York, NY, pp.16-18 (1985) and the discussion therein is incorporated herein by reference. Electron withdrawing groups include halo, including bromo, fluoro, chloro, iodo and the like; nitro, carboxy, lower alkenyl, lower alkynyl, formyl, carboxyamido, aryl, quaternary ammonium, trifluoromethyl, aryl lower alkanoyl, carbalkoxy and the like. Electron donating groups include such groups as hydroxy, lower alkoxy, including methoxy, ethoxy and the like; lower alkyl, such as methyl, ethyl, and the like; amino, lower alkylamino, di (loweralkyl) amino, aryloxy such as phenoxy, mercapto, lower alkylthio, lower alkylmercapto, disulfide (lower alkyldithio) and the like. One of ordinary skill in the art will appreciate that some of the aforesaid substituents may be considered to be electron donating or electron withdrawing under different chemical conditions. Moreover, the present invention contemplates any combination of substituents selected from the above-identified groups.

The term "halo" includes fluoro, chloro, bromo, iodo and the like.

The term "acyl" includes lower alkanoyl containing from 1 to 6 carbon atoms and may be straight chains or branched. These groups include, for example, formyl, acetyl, propionyl, butyryl, isobutyryl, tertiary butyryl, pentanoyl and hexanoyl.

As employed herein, the heterocyclic substituent contains at least one sulfur, nitrogen or oxygen ring atom, but also may include one or several of said atoms in the ring. The heterocyclic substituents contemplated by the present invention include heteroaromatics and saturated and partially saturated heterocyclic compounds. These heterocyclics may be monocyclic, bicyclic, tricyclic or polycyclic and are fused rings. They may contain up to 18 ring atoms and up to a total of 17 ring carbon atoms and a total of up to 25 carbon atoms. The heterocyclics are also intended to include the so-called benzoheterocyclics. Representative heterocyclics include furyl, thienyl, pyrazolyl, pyrrolyl, methylpyrrolyl, imidazolyl, indolyl, thiazolyl, oxazolyl, isothiazolyl, isoxazolyl, piperidyl, pyrrolinyl, piperazinyl, quinolyl, triazolyl, tetrazolyl, isoquinolyl, benzofuryl, benzothienyl, morpholinyl, benzoxazolyl, tetrahydrofuryl, pyranyl, indazolyl, purinyl, indolinyl, pyrazolindinyl, imidazolinyl, imadazolindinyl, pyrrolidinyl, furazanyl, N-methylindolyl, methylfuryl, pyridazinyl, pyrimidinyl, pyrazinyl, pyridyl, epoxy, aziridino, oxetanyl, azetidinyl, the N-oxides of the nitrogen containing heterocycles, such as the N-oxides of pyridyl, pyrazinyl, and pyrimidinyl and the like.

The preferred heterocyclics are thienyl, furyl, pyrrolyl, benzofuryl, benzothienyl, indolyl, methylpyrrolyl, morpholinyl, pyridiyl, pyrazinyl, imidazolyl, pyrimidinyl, or pyridazinyl. The preferred heterocyclic is a 5 or 6-membered heterocyclic compound. The especially preferred heterocyclic is furyl, pyridyl, pyrazinyl, imidazolyl, pyrimidinyl, or pyridazinyl. The most preferred heterocyclics are furyl and pyridyl.

The preferred compounds are those wherein n is 1, but di (n=2), tri (n=3) and tetrapeptides (n=4) are also contemplated to be within the scope of the invention.

The preferred values of R is aryl lower alkyl, especially benzyl especially those wherein the phenyl ring thereof is unsubstituted or substituted with electron donating groups or electron withdrawing groups, such as halo (e.g., F).

The preferred R₁ is H or lower alkyl. The most preferred R₁ group is methyl.

The preferred electron donating substituents or/and electron withdrawing substituents are halo, nitro, alkanoyl, formyl, arylalkanoyl, aryloyl, carboxyl, carbalkoxy, carboxamido, cyano, sulfonyl, sulfoxide, heterocyclic, guanidine, quaternary ammonium, lower alkenyl, lower alkynyl, sulfonium salts, hydroxy, lower alkoxy, lower alkyl, amino, lower alkylamino, di(loweralkyl)amino, amino lower alkyl, mercapto, mercaptoalkyl, alkylthio, and alkyldithio. The term "sulfide" encompasses mercapto, mercapto alkyl and alkylthio, while the term disulfide encompasses alkyldithio. Especially preferred electron donating or/and electron withdrawing groups are halo or lower alkoxy, most preferred are fluoro or methoxy. These preferred substituents may be substituted on any one of R, R₁, R₂, R₃, R₄, R₅ R₆, R'₆, R₇, R₈ or R₅₀ as defined herein.

The ZY groups representative of R₂ and R₃ include hydroxy, alkoxy, such as methoxy, ethoxy, aryloxy, such as phenoxy; thioalkoxy, such as thiomethoxy, thioethoxy; thioaryloxy such as thiophenoxy; amino; alkylamino, such as methylamino, ethylamino; arylamino, such as anilino; lower dialkylamino, such as, dimethylamino; trialkyl ammonium salt, hydrazino; alkylhydrazino and arylhydrazino, such as N-methylhydrazino, N-phenylhydrazino, carbalkoxy hydrazino, aralkoxycarbonyl hydrazino, aryloxycarbonyl hydrazino, hydroxylamino, such as N-hydroxylamino (-NH-OH), lower alkoxy amino [(NHOR₁₈) wherein R₁₈ is lower alkyl], N-lower alkylhydroxyl amino [(NR₁₈)OH wherein R₁₈ is lower alkyl], N-lower alkyl-O-lower alkylhydroxyamino, i.e., [N(R₁₈)OR₁₉ wherein R₁₈ and R₁₉ are independently lower alkyl], and o-hydroxylamino (-O-NH₂); alkylamido such as acetamido; trifluoroacetamido; lower alkoxyamino, (e.g., NH(OCH₃); and heterocyclicamino, such as pyrazoylamino.

The preferred heterocyclic groups representative of R₂ and R₃ are monocyclic 5- or 6-membered heterocyclic moieties of the formula: 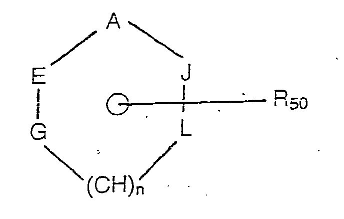 or those corresponding partially or fully saturated form thereof wherein n is 0 or 1; and
R₅₀ is H or an electron withdrawing group or electron donating group;
A, E, L, J and G are independently CH, or a heteroatom selected from the group consisting of N, O, S;
but when n is 0, G is CH, or a heteroatom selected from the group consisting of NH, O and S with the proviso that at most two of A, E, L, J and G are heteroatoms.

When n is 0, the above heteroaromatic moiety is a five membered ring, while if n is 1, the heterocyclic moiety is a six membered monocyclic heterocyclic moiety. The preferred heterocyclic moieties are those aforementioned heterocyclics which are monocyclic.

If the ring depicted hereinabove contains a nitrogen ring atom, then the N-oxide forms are also contemplated to be within the scope of the invention.

When R₂ or R₃ is a heterocyclic of the above formula, it may be bonded to the main chain by a ring carbon atom. When n is 0, R₂ or R₃ may additionally be bonded to the main chain by a nitrogen ring atom.

Other preferred moieties of R₂ and R₃ are hydrogen, aryl, e.g., phenyl, aryl alkyl, e.g., benzyl and alkyl.

It is to be understood that the preferred groups of R₂ and R₃ may be unsubstituted or substituted with electron donating or electron withdrawing groups. It is preferred that R₂ and R₃ are independently hydrogen, lower alkyl, which is either unsubstituted or substituted with an electron withdrawing group or an electron donating group, such as lower alkoxy (e.g., methoxy, ethoxy, and the like), N-hydroxylamino, N-lower alkylhydroxyamino, N-loweralkyl-O-loweralkyl and alkylhydroxyamino.

It is preferred that one of R₂ and R₃ is hydrogen.

It is preferred that n is one.

It is more prefered that n=1 and one of R₂ and R₃ is hydrogen. It is especially preferred that in this embodiment, R₂ is hydrogen and R₃ is lower alkyl or ZY; Z is O, NR₄ or PR₄; Y is hydrogen or lower alkyl; ZY is NR₄NR₅R₇, NR₄OR₅, ONR₄R₇,

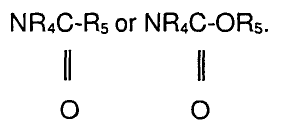

In another especially preferred embodiment, n=1, R₂ is hydrogen and R₃ is lower alkyl which may be substituted or unsubstituted with an electron donating or electron withdrawing group, NR₄OR₅, or ONR₄R₇,

In yet another especially preferred embodiment, n=1, R₂ is hydrogen and R₃ is lower alkyl which is unsubstituted or substituted with hydroxy or loweralkoxy, NR₄OR₅ or ONR₄R₇, wherein R₄, R₅ and R₇ are independently hydrogen or lower alkyl, R is aryl lower alkyl, which aryl group may be unsubstituted or substituted with an electron withdrawing group and R₁ is lower alkyl. In this embodiment it is most preferred that aryl is phenyl, which is unsubstituted or substituted with halo.

It is preferred that R₂ is hydrogen and R₃ is hydrogen, an alkyl group which is unsubstituted or substituted by at least an electron donating or electron withdrawing group or ZY. In this preferred embodiment, it is more preferred that R₃ is hydrogen, an alkyl group such as methyl, which is unsubstituted or substituted by an electron donating group, or NR₄OR₅ or ONR₄R₇, wherein R₄, R₅ and R₇ are independently hydrogen or lower alkyl. It is preferred that the electron donating group is lower alkoxy, and especially methoxy or ethoxy.

It is preferred that R₂ and R₃ are independently hydrogen, lower alkyl, or ZY;
Z is O, NR₄ or PR₄;
Y is hydrogen or lower alkyl or
   ZY is NR₄R₅R₇, NR₄OR₅, ONR₄R₇,

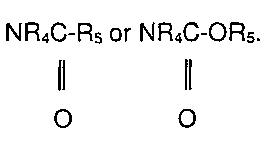

It is also preferred that R is aryl lower alkyl. The most preferred aryl for R is phenyl. The most preferred R group is benzyl. In a preferred embodiment, the aryl group may be unsubstituted or substituted with an electron donating or electron withdrawing group. If the aryl ring in R is substituted, it is most preferred that it is substituted with an electron withdrawing group, especially on the aryl ring. The most preferred electron withdrawing group for R is halo, especially fluoro.

The preferred R₁ is loweralkyl, especially methyl.

It is more preferred that R is aryl lower alkyl and R₁ is lower alkyl.

Further preferred compounds are compounds of Formula (Ib) wherein n is 1; R₂ is hydrogen; R₃ is hydrogen, a lower alkyl group, especially methyl which is substituted by an electron donating or electron withdrawing group or ZY; R is aryl, aryl lower alkyl, such as benzyl, wherein the aryl group is unsubstituted or substituted with an electron donating or electron withdrawing group and R₁ is lower alkyl. In this embodiment, it is more preferred that R₃ is hydrogen, a lower alkyl group, especially methyl, which may be substituted by electron donating group, such as lower alkoxy, (e.g., methoxy, ethoxy and the like), NR₄OR₅ or ONR₄R₇ wherein these groups are defined hereinabove.

The most preferred compounds utilized are those of the Formula (IIb): 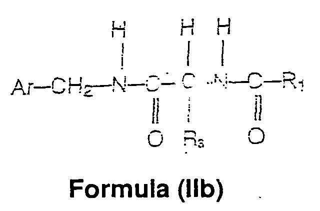 wherein
Ar is aryl, especially phenyl, which is unsubstituted or substituted with at least one electron donating group or electron withdrawing group, especially halo,
R₁ is lower alkyl, especially containing 1-3 carbon atoms; and
R₃ is as defined herein, but especially hydrogen, loweralkyl, which is unsubstituted or substituted by at least an electron donating group or electron withdrawing group or ZY. It is even more preferred that R₃ is, in this embodiment, hydrogen, an alkyl group which is unsubstituted or substituted by an electron donating group, NR₄OR₅ or ONR₄R₇. It is most preferred that R₃ is CH₂-Q, wherein Q is lower alkoxy, especially containing 1-3 carbon atoms; NR₄OR₅ or ONR₄R₇ wherein R₄ is hydrogen or alkyl containing 1-3 carbon atoms, R₅ is hydrogen or alkyl containing 1-3 carbon atoms, and R₇ is hydrogen or alkyl containing 1-3 carbon atoms.

The most preferred R₁ is CH₃. The most preferred R₃ is CH₂-Q, wherein Q is methoxy.
The most preferred aryl is phenyl. The most preferred halo is fluoro.
The most preferred compound includes:
(R)-2-acetamido-N-benzyl-3-methoxy-propionamide,
O-methyl-N-acetyl-D-serine-m-fluorobenzyl-amide;
O-methyl-N-acetyl-D-serine-p-fluorobenzyl-amide;
N-acetyl-D-phenylglycine benzylamide;
D-1,2-(N,O-dimethylhydroxylamino)-2-acetamide acetic acid benzylamide;
D-1,2-(O-methylhydroxylamino)-2-acetamido acetic acid benzylamide.

It is to be understood that the various combinations and permutations of the Markush groups of R₁, R₂, R₃, R and n described herein are contemplated to be within the scope of the present invention. Moreover, the present invention also encompasses compounds and compositions which contain one or more elements of each of the Markush groupings in R₁, R₂, R₃, n and R and the various combinations thereof. Thus, for example, the present invention contemplates that R₁ may be one or more of the substituents listed hereinabove in combination with any and all of the substituents of R₂, R₃, and R with respect to each value of n.

The compounds utilized in the present invention may contain one or more asymmetric carbons and may exist in racemic and optically active forms. The configuration around each asymmetric carbon can be either the D or L form. It is well known in the art that the configuration around a chiral carbon atoms can also be described as R or S in the Cahn-Prelog-Ingold nomenclature system. All of the various configurations around each asymmetric carbon, including the various enantiomers and diastereomers as well as racemic mixtures and mixtures of enantiomers, diastereomers or both are contemplated by the present invention.

In the principal chain, there exists asymmetry at the carbon atom to which the groups R₂ and R₃ are attached. When n is 1, the compounds of the present invention is of the formula 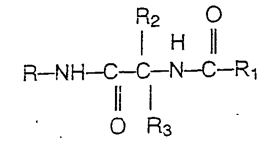 wherein R, R₁, R₂, R₃, R₄, R₅, R₆, R'₆, R₇, R₈, R₅₀ Z and Y are as defined previously.

As used herein, the term configuration shall refer to the configuration around the carbon atom to which R₂ and R₃ are attached, even though other chiral centers may be present in the molecule. Therefore, when referring to a particular configuration, such as D or L, it is to be understood to mean the D or L stereoisomer at the carbon atom to which R₂ and R₃ are attached. However, it also includes all possible enantiomers and diastereomers at other chiral centers, if any, present in the compound.

The compounds of the present invention are directed to all the optical isomers, i.e., the compounds of the present invention are either the L-stereoisomer or the D-stereoisomer (at the carbon atom to which R₂ and R₃ are attached). These stereoisomers may be found in mixtures of the L and D stereoisomer, e.g., racemic mixtures. The D stereoisomer is preferred.

Depending upon the substituents, the present compounds may form addition salts as well. All of these forms are contemplated to be within the scope of this invention including mixtures of the stereoisomeric forms

The preparation of the utilized compounds are described in U.S. Patent Nos. 5,378,729 and 5,773.475, the contents of both of which are incorporated by reference.

The compounds utilized in the present invention are useful as such as depicted in the Formula (Ib) or can be employed in the form of salts in view of its basic nature by the presence of the free amino group. Thus, the compounds of Formula (Ib) forms salts with a wide variety of acids, inorganic and organic, including pharmaceutically acceptable acids. The salts with therapeutically acceptable acids are of course useful in the preparation of formulation where enhanced water solubility is most advantageous.

These pharmaceutically acceptable salts have also therapeutic efficacy. These salts include salts of inorganic acids such as hydrochloric, hydroiodic, hydrobromic, phosphoric, metaphosphoric, nitric acid and sulfuric acids as well as salts of organic acids, such as tartaric, acetic, citric, malic, benzoic, perchloric, glycolic, gluconic, succinic, aryl sulfonic, (e.g., p-toluene sulfonic acids, benzenesulfonic), phosphoric, malonic, and the like.

It is preferred that the compound utilized in the present invention is used in therapeutically effective amounts.

The physician will determine the dosage of the present therapeutic agents which will be most suitable and it will vary with the form of administration and the particular compound chosen, and furthermore, it will vary with the patient under treatment, the age of the patient, the type of malady being treated. He will generally wish to initiate treatment with small dosages substantially less than the optimum dose of the compound and increase the dosage by small increments until the optimum effect under the circumstances is reached. When the composition is administered orally, larger quantities of the active agent will be required to produce the same effect as a smaller quantity given parenterally. The compounds are useful in the same manner as comparable therapeutic agents and the dosage level is of the same order of magnitude as is generally employed with these other therapeutic agents.

In a preferred embodiment, the compounds utilized are administered in amounts ranging from about 1 mg to about 100 mg per kilogram of body weight per day. This dosage regimen may be adjusted by the physician to provide the optimum therapeutic response. For example, several divided doses may be administered daily or the dose may be proportionally reduced as indicated by the exigencies of the therapeutic situation. The compounds of Formula (Ib) may be administered in a convenient manner, such as by oral, intravenous (where water soluble), intramuscular, intrathecal or subcutaneous routes.

The compounds of Formula (Ib) may be orally administered, for example, with an inert diluent or with an assimilable edible carrier, or it may be enclosed in hard or soft shell gelatin capsules, or it may be compressed into tablets, or it may be incorporated directly into the fool of the diet. For oral therapeutic administration, the active compound of Formula (Ib) may be incorporated with excipients and used in the form of ingestible tablets, buccal tablets, troches, capsules, elixirs, suspensions, syrups, wafers, and the like. Such compositions and preparations should contain at least 1 % of active compound of Formula (Ib). The percentage of the compositions and preparations may, of course, be varied and may conveniently be between about 5 to about 80 % of the weight of the unit. The amount of active compound of Formula (Ib) in such therapeutically useful compositions is such that a suitable dosage will be obtained. Preferred compositions or preparations according to the present invention contains between about 10 mg and 6 g active compound of Formula (Ib).

The tablets, troches, pills, capsules and the like may also contain the following: A binder such as gum tragacanth, acacia, corn starch or gelatin; excipients such as dicalcium phosphate; a disintegrating agent such as corn starch, potato starch, alginic acid and the like; a lubricant such as magnesium stearate; and a sweetening agent such as sucrose, lactose or saccharin may be added or a flavoring agent such as peppermint, oil of wintergreen, or cherry flavoring. When the dosage unit form is a capsule, it may contain, in addition to materials of the above type, a liquid carrier.

Various other materials may be present as coatings or otherwise modify the physical form of the dosage unit. For instance, tablets, pills, or capsules may be coated with shellac, sugar or both. A syrup or elixir may contain the active compound, sucrose as a sweetening agent, methyl and propylparabens as preservatives, a dye and flavoring such as cherry or orange flavor. Of course, any material used in preparing any dosage unit form should be pharmaceutically pure and substantially non-toxic in the amounts employed. In addition, the active compound may be incorporated into sustained-release preparations and formulations. For example, sustained release dosage forms are contemplated wherein the active ingredient is bound to an ion exchange resin which, optionally, can be coated with a diffusion barrier coating to modify the release properties of the resin.

The active compound may also be administered parenterally, rectally (e.g. suppository, gel, liquid, etc) or intraperitoneally. Dispersions can also be prepared in glycerol, liquid, polyethylene glycols, and mixtures thereof and in oils. Under ordinary conditions of storage and use, these preparations contain a preservative to prevent the growth of microorganisms.

The pharmaceutical forms suitable for injectable use include sterile aqueous solutions (where water soluble) or dispersions and sterile powders for the extemporaneous preparation of sterile injectable solutions or dispersions. In all cases the form must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyethylene glycol, and the like), suitable mixtures thereof, and vegetable oils. The proper fluidity can be maintained, for example, by the use of a coating such as lecithin, by the maintenance of the required particle size in the case of dispersions and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminium monostearate and gelatin.

Sterile injectable solutions are prepared by incorporating the active compound in the required amount in the appropriate solvent with various of the other ingredients enumerated above, as required, followed by filtered sterilization. Generally, dispersions are prepared by incorporating the various sterilized active ingredient into a sterile vehicle which contains the basic dispersion medium and the required other ingredients from those enumerated above. In the case of sterile powders for the preparation of sterile injectable solutions, the preferred methods of preparation are vacuum drying the freeze-drying technique plus any additional desired ingredient from previously sterile-filtered solution thereof.

As used herein, "pharmaceutically acceptable carrier" includes any and all solvents, dispersion media, coatings, antibacterial and antifungal agent, isotonic and absorption delaying agents for pharmaceutical active substances as well known in the art. Except insofar as any conventional media or agent is incompatible with the active ingredient, its use in the therapeutic compositions is contemplated. Supplementary active ingredients can also be incorporated into the compositions.

It is especially advantageous to formulate parenteral compositions in dosage unit form or ease of administration and uniformity of dosage. Dosage unit form as used herein refers to physically discrete units suited as unitary dosages for the mammalian subjects to be treated; each unit containing a predetermined quantity of active material calculated to produce the desired therapeutic effect in association with the required pharmaceutical carrier. The specifics for the novel dosage unit forms of the invention are dictated by and directly dependent on (a) the unique characteristics of the active material an the particular therapeutic effect to be achieved, and (b) the limitations inherent in the art of compounding such as active material for the treatment of disease in living subjects having a diseased condition in which bodily health is impaired as herein disclosed in detail.

The principal active ingredient is compounded for convenient and effective administration in effective amounts with a suitable pharmaceutically acceptable carrier in dosage unit form as hereinbefore described. A unit dosage form can, for example, contain the principal active compound in amounts ranging from about 10 mg to about 6 g. Expressed in proportions, the active compound is generally present in from about 1 to about 750 mg/ml of carrier. In the case of compositions containing supplementary active ingredients, the dosages are determined by reference to the usual dose and manner of administration of the said ingredients.

As used herein the term "patient" or "subject" refers to a warm blooded animal, and preferably mammals, such as, for example, cats, dogs, horses, cows, pigs, mice, rats and primates, including humans. The preferred patient is humans.

The term "treat" refers to either relieving the *status epilepticus* associated with a disease or condition or alleviating the patient's disease or condition.

The compounds of the present invention are administered to a patient suffering from *status epilepticus* or related conditions. These amounts are equivalent to the therapeutically effective amounts described hereinabove.

The used substance was SPM 927 which is the synonym for Harkoseride. The standard chemical nomenclature is (R)-2-acetamide-N-benzyl-3-methoxypropionamide.

The invention is further demonstrated by the following Example and Figures.

### Figure and Table Legend

**Figure 1** demonstrates the effects of SPM 927, diazepam and phenytoin on SSSE.
**Figure 2** demonstrates the effects of SPM 927 and fosphenytoin (F-PHT) on SSSE (**A**: seizure duration, **B**: number of spikes/10 min).
**Table 1** shows the effects of SPM 927 on hippocampal damage folloing SSSE.
**Table 2** shows the effects of SPM 927 on chemically induced SSSE.

### Example 1

### SPM 927 is anti-convulsive and neuroprotective in rat models for self-sustaining status epilepticus.

SPM 927 shows a more potent and effective anticonvulsant profile in animal models compared to other antiepileptic drugs (e.g. phenytoin, carbamazepine). SPM 927 is available as both an oral and an intravenous formulation. It was, therefore, of interest to determine the potential efficacy of SMP 927 in animal models of self-sustaining status epilepticus (SSSE).

### Experiment 1

### SSSE after brief electrical stimulation of the perforant path

**Animals:** The experiments were performed on male Wistar rats, 280-300g (Simonsen Labs, CA). Animals were kept at a constant room temperature with 12 hours artificial dark-light cycle with free access to standard diet and tap water.

**Surgery:** Under ketamine (60 mg/kg)/xylazine (15 mg/kg) anesthesia, animals were implanted with a bipolar stimulating electrode into the angular bundle of the perforant path (4.5 mm to left lambda, 1 mm anterior to lambdoid fissure) and a bipolar recording electrode into the granule cell layer of the ipsilateral dentate gyrus (3.5 mm anterior to lambda, 2.2 mm left to saggital fissure). Depth of both electrodes was optimized by monitoring the amplitude and waveform of the population spike evoked from the dentate gyrus by stimuli delivered through the perforant path (single square wave monophasic stimuli, 20 V, 0.1 ms delivered every 10 s).

**Induction of self-sustaining *status epilepticus* (SSSE):** Seven days after surgery, animals were connected to a Cardionics 16 channel preamplifier, which was connected to the IBM computer. After 5 minutes of baseline EEG recording, animals were stimulated in the awake state for 60 minutes (Experiment 1 a) or 30 minutes (Experiments 1b and 1 c) with 10 s 20 Hz trains (1 ms square wave, 20 V) delivered every minute, together with 2 Hz continuous stimulation with the same parameters.

**EEG monitoring and analysis:** EEG from the dentate gyrus was monitored and recorded by means of commercial software (*Monitor 8.1* from Stellate Systems). The software was configured for automatic detection and saving of spikes and seizures. Monitoring was performed for 24 hrs. EEG was analyzed off line by means of the same software. Spike distribution was presented as number of spikes per 10 min epoch, and the cumulated duration of seizures for 24 hrs of monitoring were calculated. Statistical analysis was performed by means of T-test. A p value less than 0.05 was considered statistically significant.

**Drug administration:** SPM 927 (50 mg/kg, 5 animals), phenytoin (PHT) (50 mg/kg), Fosphenytoin (F-PTH Porte-Davis, 50 mg/kg of phenytoin equivalent, 5 animals), diazepam (DZP) (10mg/kg) or vehicle (VEH, 10% DMSO, 6 animals) were given i.v. 10 min after the end of perforant path stimulation in a volume of 1 ml/kg.

**Collection of tissue:** Brains were collected 72 hours after SSSE. This was accomplished by transcardial perfusion with first heparinized normal saline for about 2 minutes to largely clear the vasculature of blood and second by phosphate buffered formaldehyde (10% formaldehyde at pH 7.4) for 10 to 15 min (approximately 50 to 60 ml). Carcasses were placed in the refrigerator overnight to further firm the tissue and the brains were dissected from the heads the following day.

**Preparation of brain sections for light microscopy:** After removal, the brains were passed through a graded series of alcohol and clearing agent (Hemo-De, Fisher) and then embedded in paraffin. Serial 8 micron-thick coronal sections were cut from the embedded tissue at approximately 100 micron intervals and float mounted onto microscope slides. The sections were stained with hematoxylin and eosin and coverslipped.

**Selection of sections for counting:** Selection of sections for counting was controlled by the stereotaxic atlas for rats (Paxinos and Watson). The 3 sections selected for quantitation contained dorsal hippocampus and were 1.6 ±0.05mm posterior to bregma.

### Counting:

**Location of counting fields ―** damage to the pyramidal cell layer was assessed by counting damaged neurons in the subiculum, CA1, CA3, CA3c and the hilus in the dorsal hippocampus. The neurons were assessed individually from the beginning of the subiculum to the end of the CA3c region using an Olympus Provis Ax 70 microscope at a magnification of 40x. The CA3-CA3c transition was defined by a line connecting the extremeties of the blades of the dentate gyrus. The hilus was defined as the space located between the blades of the dentate gyrus, excluding the line of CA3c pyramids.

**Criteria for differentiation of damaged versus non-damaged neurons -** neurons were counted only if the majority of the nucleus was present in the section. Damaged neurons were counted if they were distinctly eosinophilic or distinctly pyknotic or both. All other cells with most of the nucleus present in the section (and counting field) were counted as not damaged. Each brain was assessed on left side and right side individually and also combined. The counting fields were individually divided into subiculum, CA1, CA3, CA3c and hilus.

**Qualitative scale for assessing damage on the ipsi- and contralateral side of the stimulation.**
0 = no damaged neurons
1 = fewer than 5% damaged neurons
2 = 5 to 10% damaged neurons
3 = 10 to 25% damaged neurons
4 = 25 to 50% damage neurons
5 = greater than 50% damaged neurons.

### Results:

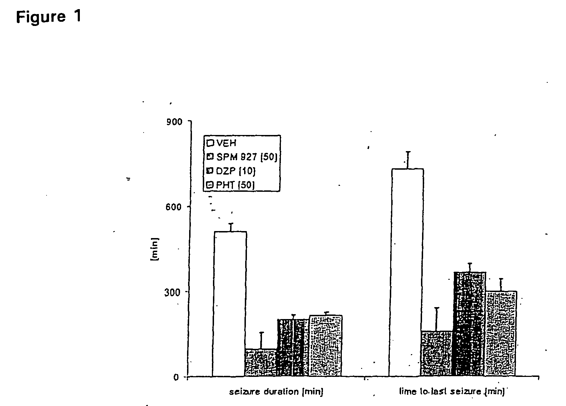

**Experiment 1a:** Compared to control animals, SPM 927, DZP and PHT potently and significantly reduced cumulative seizure duration over the 24h period and the time to the last seizure. SPM 927 at the dose tested was somewhat more efficacious on both parameters when compared to DZP and PHT (Fig. 1).

### Experiment 1b: Effects of SPM 927 and fosphenytoin (F-PHT) on SSSE

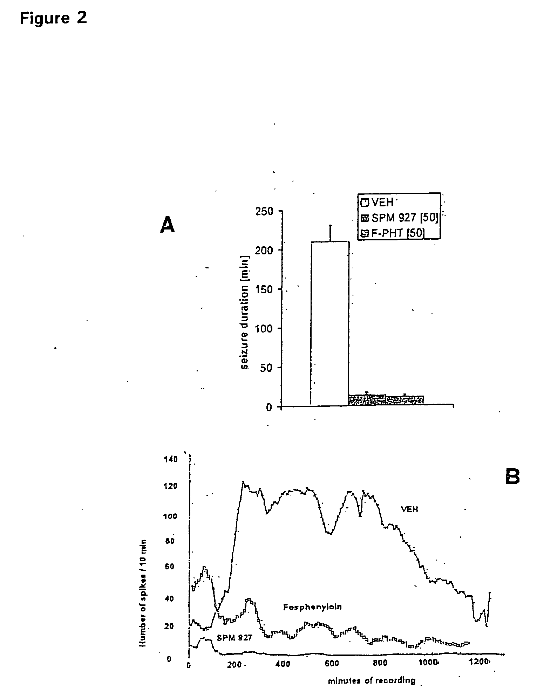

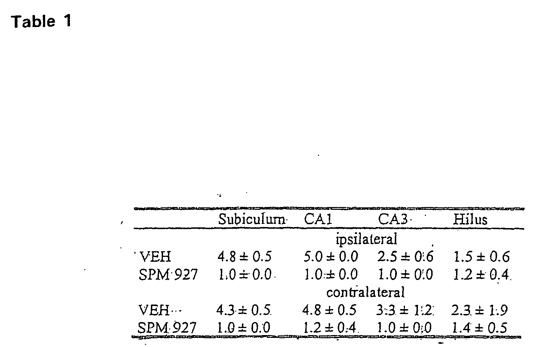

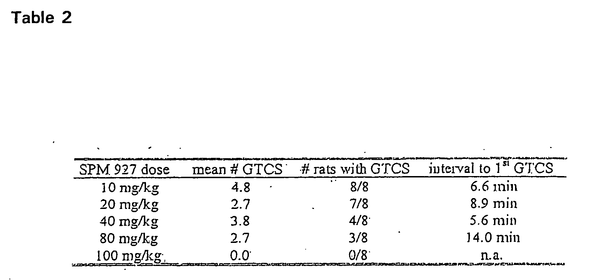

- **- Control of animals:** SSSE consisted of recurrent limbic seizures for up to 24hrs after the end of stimulation (Fig. 2A). Nearly continuous spikes were recorded between seizures (Fig. 2B).
- **SPM 927:** Seizures stopped within 15 min after injection and did not recur over the next 24 hrs (Fig. 2A). Spike frequency was significantly decreased and only a few single spikes were recorded for about 12 hrs (Fig. 2B).
- **- Fosphenytoin:** Seizures stopped within 15 min (Fig. 2A). Spike frequency was significantly lower than in control animals but significantly higher than in SPM 927 treated rats (Fig. 2B).

### Experiment 1c: Effects of SPM 927 on hippocampal damage following SSSE

Table 1 shows that untreated rats suffered massive neuronal injury in CA1 and subiculum, and moderate injury to hilus and CA3. Animals treated with SPM 927 showed only an occasional injured neuron in any hippocampal field. The protection was statistically significant in the subiculum, CA1 and CA3.

### Experiment 2

### Chemically induced SSSE (homocysteine injection)

Male Sprague-Dawley rats were prepared surgically with cobalt powder on the left frontal cortical surface.

**Recording and stimulation protocol:** four epidural recording electrodes arranged in a square grid (5mm) over the cobalt lesion. EEG was monitored daily beginning 4-5 days following surgery. *Status epilepticus* was induced by i.p. injection of 5.5mmol/kg homocysteine thiolactone whenever focal motor behaviour and EEG seizures were observed.

**Drug administration:** SPM 927 was administered (10-100mg/kg) immediately following the second generalized tonic-clonic seizure (GTCS) occurring after homocysteine thiolactone injection. Rats were observed for 30 min following treatment.

**Results:** Table 2 shows the mean number of GTCS occurring in the 30 min following treatment with SPM 927. The number of rats experiencing GTCS reduced in a dose-dependent fashion. In addition, SPM 927 potently reduced the mean number of GTCS and prolonged the latency to the first GTCS. The highest dose of SPM 927 completely abolished all GTCS.

### Summary and Conclusions

SPM 927 potently reduced cumulative SSSE duration after brief electrical stimulation of the perforant path in two independent experiments.

SPM 927 effect in reducing the number of spikes was significantly more potent than that of fosphenytoin.

SPM 927 dramatically reduced neuronal hippocampal damage following perforant path stimulation.

SPM 927 prevented spontaneous GTCS in the cobalt/homocysteine model of SSSE.

These results suggest that i.v. SPM 927 may be useful for the treatment of generalized convulsive *status epilepticus* or related conditions in humans or as a neuroprotective treatment before/during acute seizures, in particular during *status epilepticus* or related conditions, or during chronic seizure disorders (e.g. epilepsy), to reduce brain damage, short term memory loss, cognitive decline, additional seizures (anti-epileptogenesis), etc.

## Claims

**1.** Use of a compound having the Formula (Ib) 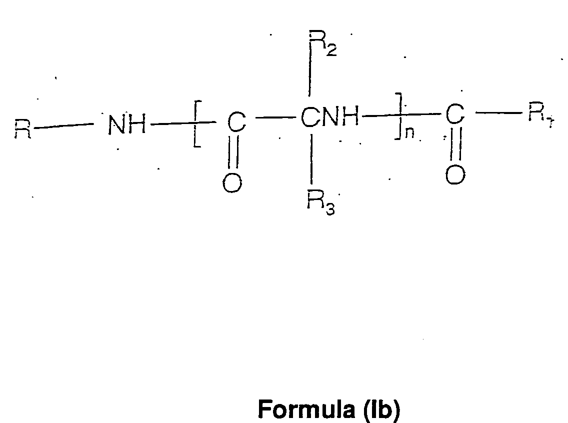 wherein
R is hydrogen, lower alkyl, lower alkenyl, lower alkynyl, aryl, aryl lower alkyl, heterocyclic, heterocyclic lower alkyl, lower alkyl heterocyclic, lower cycloalkyl or lower cycloalkyl lower alkyl, and R is unsubstituted or is substituted with at least one electron withdrawing group or electron donating group;
R₁ is hydrogen or lower alkyl, lower alkenyl, lower alkynyl, aryl lower alkyl, aryl, heterocyclic lower alkyl, lower alkyl heterocyclic, heterocyclic, lower cycloalkyl, lower cycloalkyl lower alkyl, each unsubstituted or substituted with an electron donating group or an electron withdrawing group;
R₂ and R₃ are independently hydrogen, lower alkyl, lower alkenyl, lower alkynyl, aryl lower alkyl, aryl, halo, heterocyclic, heterocyclic lower alkyl, lower alkyl heterocyclic, lower cycloalkyl, lower cycloalkyl lower alkyl, or Z-Y wherein R₂ and R₃ may be unsubstituted or substituted with at least one electron withdrawing group or electron donating group; and wherein heterocyclic in R₂ and R₃ is furyl, thienyl, pyrazolyl, pyrrolyl, methylpyrrolyl, imidazolyl, indolyl, thiazolyl, oxazolyl, isothiazolyl, isoxazolyl, piperidyl, pyrrolinyl, piperazinyl, quinolyl, triazolyl, tetrazolyl, isoquinolyl, benzofuryl, benzothienyl, morpholinyl, benzoxazolyl, tetrahydrofuryl, pyranyl, indazolyl, purinyl, indolinyl, pyrazolindinyl, imidazolinyl, imidazolindinyl, pyrrolidinyl, furazanyl, N-methylindolyl, methylfuryl, pyridazinyl, pyrimidinyl, pyrazinyl, pyridyl, epoxy, aziridino, oxetanyl, azetidinyl or, when N is present in the heterocyclic, an N-oxide thereof;
Z is O, S, S(O)ₐ, NR₄, NR₆' or PR₄ or a chemical bond;
Y is hydrogen, lower alkyl, aryl, aryl lower alkyl, lower alkenyl, lower alkynyl, halo, heterocyclic, heterocyclic lower alkyl, lower alkyl heterocyclic and Y may be unsubstituted or substituted with an electron donating group or an electron withdrawing group, wherein heterocyclic has the same meaning as in R₂ or R₃ and, provided that when Y is halo, Z is a chemical bond, or
ZY taken together is NR₄NR₅R₇, NR₄OR₅, ONR₄R₇, OPR₄R₅, PR₄OR₅, SNR₄R₇, NR₄SR₇, SPR₄R₅, PR₄SR₇, NR₄PR₅R₆, PR₄NR₅R₇, or N⁺R₅R₆R₇, 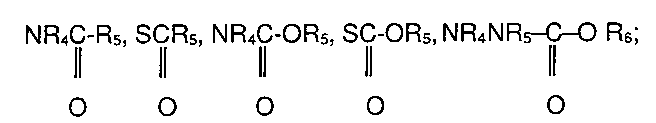
R₆' is hydrogen, lower alkyl, lower alkenyl, or lower alkynyl which may be unsubstituted or substituted with an electron withdrawing group or electron donating group;
R₄, R₅ and R₆ are independently hydrogen, lower alkyl, aryl, aryl lower alkyl, lower alkenyl, or lower alkynyl, wherein R₄, R₅ and R₆ may independently be unsubstituted or substituted with an electron withdrawing group or an electron donating group; and
R₇ is R₆ or COOR₈ or COR₈, which R₇ may be unsubstituted or substituted with an electron withdrawing group or an electron donating group;
R₈ is hydrogen or lower alkyl, or aryl lower alkyl, and the aryl or alkyl group may be unsubstituted or substituted with an electron withdrawing group or an electron donating group; and
n is 1-4; and
a is 1-3,
or of a pharmaceutically acceptable salt thereof,
for the preparation of a pharmaceutical composition for the treatment of *status epilepticus* or related conditions, e.g. acute repetitive seizures, seizure clusters, etc.

**2.** Use of a compound according to claim 1 wherein one of R₂ and R₃ is hydrogen.

**3.** Use of a compound according to claim 1 wherein n is 1.

**4.** Use of a compound according to claim 1 wherein one of R₂ and R₃ is hydrogen and n is 1.

**5.** Use of a compound according to claim 1 wherein R is aryl lower alkyl and R₁ is lower alkyl.

**6.** Use of a compound according to claim 1 wherein
R₂ and R₃ are independently hydrogen, lower alkyl, or ZY;
Z is O, NR₄ or PR₄;
Y is hydrogen or lower alkyl or
ZY is NR₄NR₅R₇, NR₄OR₅, ONR₄R₇,

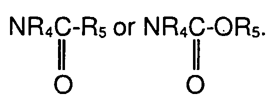

**7.** Use of a compound according to claim 4 wherein R₂ is hydrogen and and R₃ is lower alkyl, or ZY;
Z is O, NR₄ or PR₄;
Y is hydrogen or lower alkyl;
ZY is NR₄NR₅R₇, NR₄OR₅, ONR₄R₇,

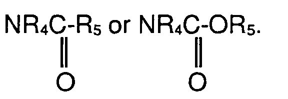

**8.** Use of a compound according to claim 4 wherein R₂ is hydrogen and R₃ is lower alkyl, which may be substituted or unsubstituted with an electron donating or electron withdrawing group, NR₄OR₅, or ONR₄R₇.

**9.** Use of a compound according to claim 4 wherein R₃ is lower alkyl which is unsubstituted or substituted with hydroxy or loweralkoxy, NR₄OR₅ or ONR₄R₇, wherein R₄, R₅ and R₇ are independently hydrogen or lower alkyl, R is aryl loweralkyl, which aryl group may be unsubstituted or substituted with an electron withdrawing group and R₁ is lower alkyl.

**10.** Use of a compound according to claim 9 wherein aryl is phenyl and is unsubstituted or substituted with halo.

**11.** Use of a compound according to claim 1 wherein the compound is
(R)-2-acetamido-N-benzyl-3-methoxy-propionamide;
O-methyl-N-acetyl-D-serine-m-fluorobenzylamide;
O-methyl-N-acetyl-D-serine-p-fluorobenzylamide;
N-acetyl-D-phenylglycinebenzylamide;
D-1,2-(N, O-dimethylhydroxylamino)-2-acetamide acetic acid benzylamide;
D-1,2-(O-methylhydroxylamino)-2-acetamido acetic acid benzylamide.

**12.** Use of a compound of claim 1 having the Formula (IIb) 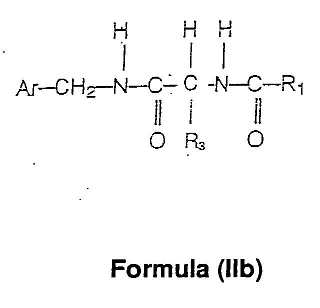 wherein
Ar is phenyl which is unsubstituted or substituted with at least one halo group;
R₃ is CH₂-Q, wherein Q is lower alkoxy containing 1-3 carbon atoms and
R₁ is lower alkyl containing 1-3 carbon atoms
or of a pharmaceutically acceptable salt thereof.

**13.** Use of a compound according to claim 12 wherein Ar is unsubstituted phenyl.

**14.** Use of a compound according to claims 12 and 13 wherein halo is fluoro.

**15.** Use of a compound according to claims 12-14 wherein R₃ is CH₂-Q, wherein Q is alkoxy containing 1-3 carbon atoms and Ar is unsubstituted phenyl.

**16.** Use of a compound of claim 1 in the R configuration having the formula 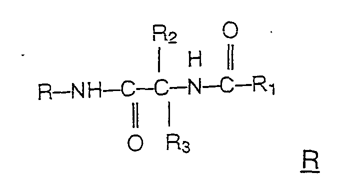 wherein
Ar is phenyl which is unsubstituted or substituted with at least one halo group;
R₃ is CH₂ -Q, wherein Q is lower alkoxy containing 1-3 carbon atoms and R₁ is methyl
or of a pharmaceutically acceptable salt thereof.

**17.** Use of the compound according to claim 16 which is substantially enantiopure.

**18.** Use of a compound according to claims 16 and 17 wherein Ar is unsubstituted phenyl.

**19.** Use of a compound according to claims 16 to 18 wherein halo is fluoro.

**20.** Use of a compound according to claims 16 to 19 wherein R₃ is CH₂-Q, wherein Q is alkoxy containing 1-3 carbon atoms and Ar is unsubstituted phenyl.

**21.** Use of a compound according to claim 1, wherein the compound of Formula (Ib) is (R)-2-Acetamido-N-benzyl-3-methoxypropionamide or a pharmaceutically acceptable salt thereof.

**22.** Use of the compound according to claim 21 which is substantially enantiopure.

**24.** Use of a compound of any one of the claims 1 to 22 for treatment of *status epilepticus* or related conditions.

**25.** Use of a compound of any one of the claims 1 to 22 using an intravenous or injectable dosage for the treatment of *status epilepticus* or related conditions.

**26.** Use of a compound of any one of the claims 1 to 22 using a rectal dosage (e.g. suppository, gel, liquid, etc) for the treatment of *status epilepticus* or related conditions.

**27.** Use of a compound of any one of the claims 1 to 22 as a neuroprotective treatment before/during acute seizures, in particular during *status epilepticus* or related conditions to reduce brain damage, short term memory loss, cognitive decline, additional seizures (anti-epileptogenesis), etc.

**28.** Use of a compound of any one of the claims 1 to 22 as a neuroprotective treatment during chronic seizure disorders (e.g. epilepsy) to reduce brain damage, short term memory loss, cognitive decline, additional seizures (anti- epileptogenesis), etc.
